(19) Europäisches Patentamt
European Patent Office
Office européen des brevets

(11) Numéro de publication: **0 133 129**
**B1**

(12) **FASCICULE DE BREVET EUROPEEN**

(45) Date de publication du fascicule du brevet: **12.10.88**

(51) Int. Cl.$^4$: **A 61 K 7/08,** A 61 K 7/13

(21) Numéro de dépôt: **84401566.9**

(22) Date de dépôt: **26.07.84**

(54) **Procédé et compositions pour la coloration des phanères.**

(30) Priorité: **28.07.83 FR 8312458**
**12.07.84 FR 8411069**

(43) Date de publication de la demande:
**13.02.85 Bulletin 85/07**

(45) Mention de la délivrance du brevet:
**12.10.88 Bulletin 88/41**

(84) Etats contractants désignés:
**AT CH DE GB LI LU NL SE**

(56) Documents cités:
**BE-A- 438 886**
**FR-A-1 016 163**
**FR-A-2 483 226**
**US-A-3 578 719**

**CHEMICAL ABSTRACTS, vol. 85, no. 6, 8 aou7t 1976, page 277, résumé no. 37085n, Columbus, Ohio, US; S.A. ZAKI: "Preparation of henna coloring shampoo using different surfactants", & BULL. FAC. PHARM., CAIRO UNIV. 1973 (Pub. 1975), 12(1), 41-5.**

(73) Titulaire: **SECTA-LABORATOIRES DE COSMETOLOGIE YVES ROCHER**
**62 Avenue d'Iéna**
**F-75116 Paris (FR)**

(72) Inventeur: **Manier, Francis**
**4 Impasse du Griffon**
**F-60940 Cinqueux (FR)**
Inventeur: **Lutz Dominique**
**22 rue Henri Bodchon**
**F-60700 Pont Sainte-Maxence (FR)**

(74) Mandataire: **Kohn, Armand**
**5 Avenue Foch**
**F-92380 Garches (FR)**

(56) References cited:
**H. MÖLLER et al.: "CHEMIE, ARBEITSVERFAHREN UND WARENKUNDE IN DER MODERNEN FRISEURPRAXIS", 3ème édition, 1973, Verlag Handwerk und Technik GmbH, Hambourg, DE**

**EP 0 133 129 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

L'invention concerne un procédé perfectionné pour la coloration de phanères, notamment poils et cheveux; elle se rapporte plus spécialement à l'utilisation de colorants ou extraits colorants, naturels, L'invention comprend également certaines compositions spéciales pour la réalisation de ce procédé de teinture.

La teinture des éléments kératiniques, et plus particulièrement celle des cheveux ou fourrures, est connue depuis l'Antiquité; elle a été réalisée, au cours des âges, par des moyens très divers, depuis l'application de sels métalliques colorés, ou celle de colorants végétaux, tels que le hénné, utilisés par les Egyptiens dès la 3ème dynastie, jusqu'aux colorants de synthèse, pour la plupart de structure aminique, employés aux temps modernes. Malgré cette abondance de la technique antérieure, le problème d'obtention d'une bonne teinture, stable, non toxique, ne pouvant pas induire des réactions allergiques, reste posé, tant pour le traitement de poils, de fourrures, que—et surtout—en ce qui concerne la teinture de cheveux vivants ou sous forme de postiches. Il s'est avéré en effet que les anciennes formules non seulement sont d'une application malaisée et souvent longue, mais encore conduisent-elles à des teintures capillaires irrégulières et peu stables. Quant aux colorants synthétiques, généralement à base d'amines aromatiques, on en connaît à présent les dangers au point de vue sanitaire. On sait notamment que l'oxydation in situ de ces colorants, avec de l'eau oxygénée et addition d'ammoniaque, dégage une odeur peu agréable et comporte des risques certains de réactions allergiques.

Pour toutes ces raisons, la tendance actuelle consiste à revenir aux extraits colorants naturels, non nocifs, en les fixant d'une façon nouvelle, afin d'obtenir une coloration stable, de nuance voulue, sans manipulation compliquée et longue. C'est ainsi que l'on a proposé récemment, par exemple dans la publication du brevet français 2 500 748, l'utilisation de colorants naturels, extraits ou synthétisés, mélangés à des poudres épuisées de végétaux, et non épuisées ainsi qu'à des diluants solides. Ces compositions, présentées sous la forme de poudre, sont diluées à l'eau au moment de l'emploi, en vue de la formation d'une pâte, de viscosité s'échelonnant entre 1,3 et 2,7 Pas (13 et 27 poises), au moment de l'emploi. Ce procédé comporte la nécessité d'emploi de moyens mécaniques pour la préparation de la pâte à appliquer, ce qui présente déjà une sujétion dans l'application artisanale, notamment chez les coiffeurs. En outre, les pâtes, suivant cette technique, doivent présenter, dans beaucoup de cas, une réaction basique, à savoir un pH de l'ordre de 9 à 9,8, dont on sait les inconvénients pour les phanères et le cuir chevelu. De plus, cette application de l'art antérieur implique de fortes quantités de colorants ou d'extraits colorants, pour ne donner finalement qu'une modification assez faible du coloris des cheveux; d'autre part, les teintures ainsi obtenues, sont peu solides.

La présente invention apporte, sur la technique connue, une amélioration marquée en ce qu'elle permet d'obtenir la fixation solide d'un ou de plusieurs colorants naturels sur des substances kératiniques, plus particulièrement sur les cheveux ou poils, en un temps bien plus court qu'il fallait jusqu'à présent à la teinture au moyen de colorants ou extraits de colorants naturels. Un autre avantage de l'invention réside en ce qu'il suffit de colorants en plus faibles quantités qu'auparavant, pour obtenir des nuances d'intensités voulues. Ainsi, peut-on obtenir le coloris désiré et stable en un temps sensiblement égal à celui que demandent les applications des colorants synthétiques; cette durée peut être de l'ordre de 40 à 50 minutes, donc beaucoup plus courte que dans le cas des applications d'extraits naturels, selon les techniques anciennes, qui exigent jusqu'à 2 h lorsqu'il s'agit d'obtenir des nuances fortes. L'invention procure également une amélioration en ce qu'elle peut et doit comporter des pH, dans la composition appliquée, généralement inférieurs à 7 ou tout au plus atteignant 8. Le nouveau procédé suivant l'invention, est caractérisé en ce qu'il est effectué en deux étapes successives, dont la première consiste en un traitement des phanères avec une solution renfermant au moins deux composés tensioactifs, non ioniques, de nature chimique différente, et un ou plusieurs sels de métaux des groupes II à V de la Classification Périodique des Eléments, sans colorant, tandis que la seconde étape comprend l'application aux phanères d'une solution ou dispersion qui contient le ou les colorants naturels, au moins un composé tensioactif non ionique, un ou plusieurs agents tensioactifs non ioniques et/ou anioniques, un alcool en $C_1$ à $C_5$, un glycol ou glycérol et un acide gras en $C_8$ à $C_{22}$, un glycéride ou savon d'un tel acide gras.

De préférence, la solution ou dispersion de colorants contient des épaississants pour faciliter le maintien en place de la teinture.

Conformément à l'invention, la première solution de lavage et de traitement est maintenue en contact avec les phanères pendant 3 à 20 minutes, selon la nature des poils ou cheveux, et en particulier selon leur état graisseux. Aucun traitement n'étant nécessaire entre les deux étapes, sauf un rinçage à l'eau tiède, la solution ou dispersion de teinture est appliquée pendant 3 à 30 minutes, de sorte que le traitement total en les deux étapes dure en moyenne 10 à 60 minutes, et le plus souvent 40 à 50 min. La teinture est également suivie d'un rinçage à l'eau tiède.

Afin de faciliter la suite de la présente description, on y désignera, de la façon suivante, les différents produits utilisés dans les compositions servant à la réalisation du procédé.

A—Dans la solution de traitement préalable, ou lotion,

$T_1$—composé tensio-actif non ionique de la classe des alkyle aryle éthers polyalkoxylés;

$T_2$—agent tensio-actif non ionique, appartenant à une classe différente de celle de $T_1$;

M—sel ou complexe d'un cation métallique;
R—acide organique soluble dans l'eau.

B—Dans la solution ou suspension colorante, c'est-à-dire dans la teinture proprement dite:

$T_3$—composé tensio-actif non ionique, du type alkyle aryle éthers polyalkoxylés, qui peut être le même que $T_1$ ou différent;
$T_4$—composé tensio-actif anionique ou non ionique, différent de $T_3$;
P—alcool de faible masse moléculaire ou polyol dérivé d'un alcool en $C_2$ ou $C_3$;
G—acide gras ou savon d'un tel acide;
E—éventuellement aussi un épaississant;
C—colorant naturel pouvant se présenter sous sa forme pure, sous celle d'un extrait ou de parties de plantes le contenant.

Ce qui précède définit le principe du procédé et des compositions suivant l'invention. Dans ce qui suit, on trouvera des indications plus détaillées sur chacun des composants indiqués.

$T_1$—peut comprendre un ou plusieurs agents tensio-actifs, non ioniques, constitués par des alkyle aryle polyalkoxylés et plus particulièrement polyéthoxylés. Conviennent notamment les compose du type

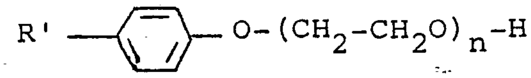

R' étant un alkyle linéaire ou ramifié en $C_1$ à $C_{20}$ et plus particulièrement en $C_4$ à $C_{12}$, tandis que n est d'au moins 5 et de préférence 6 à 15. La concentration totale en ces agents, dans la lotion aqueuse, est généralement de 20 à 80 g/l et, de préférence, 40 à 60 g/l.

$T_2$ représente un ou plusieurs composés tensioactifs, également non ioniques sans groupes aryliques, de nature différente de celle de $T_1$; ils peuvent être constitués par des moussants, détergents ou mouillants usuels dans les traitements capillaires. Tels sont par exemple des alkylamides dérivés des acides gras an $C_8$ à $C_{20}$ d'origine végétale ou animale; conviennent plus particulièrement des alcanolamides correspondants, dérivés en particulier des mono-, di- ou triéthanolamines ou propanolamines; d'autres agents $T_2$ peuvent être des esters gras du sorbitane (ou anhydro-1,4 d · sorbitol) polyéthoxylé; il s'agit principalement des esters dont l'acide gras est en $C_8$ à $C_{20}$ et le nombre de groupes éthoxy compris entre 4 et 20.

D'autres $T_2$ sont des esters gras de di- ou trialcool, par exemple ceux des acides en $C_8$ à $C_{20}$ de glycols ou de glycérol, alcools gras polyalkoxylés, en particulier alcool primaire en $C_{12}$ à $C_{15}$ ou secondaire en $C_{11}$ à $C_{15}$ avec 2 à 15 motifs d'oxyde d'éthylène par molécule; acides gras alkoxylés, notamment acides en $C_6$ à $C_{12}$ avec une chaîne oxyéthylée ayant 4 à 20 atomes d'oxygène. Parmi d'autres composés $T_2$, on peut citer des esters d'un polyéthylène glycol avec des acides gras, des produits de condensation de l'oxyde d'éthylène avec des amines grasses ou avec des acides gras, ainsi que des copolymères de l'oxyde d'éthylène avec du polyoxypropylène.

Selon la nature et le pouvoir moussant ou mouillant du tensioactif $T_2$ employé, la teneur en ce ou ces composés, dans la lotion, doit être de 5 à 30 g et, de préférence, de 8 à 20 g/l.

M, qui désigne le ou les sels métalliques utilisés, dérive des métaux non toxiques, généralement bi-ou trivalents. Ces métaux appartenant aux groupes II à V de la Classification Périodique des Eléments. L'aluminium est préféré, parce qu'il donne le nombreux sels et complexes non toxiques. Les anions de ses sels sont également choisis parmi ceux qui ne présentent aucune nocivité; on peut ainsi employer des acétates, acétoborates, acétotartrates, sulfates, boroformiates, borotanno tartrate, chloro alcoolates, chlorohydroxy borates, lactates, glycinates, dihydroxy-acétates, propionates, butyrates, alcane-polysulfonates, méthionates, β-naphtol disulfonates. De tels sels ou complexes d'aluminium sont particulièrement utiles; on peut par exemple utiliser des sels d'allantoïne et d'aluminium, des sels mixtes d'Al et de Mg, des amino-acides aliphatiques, des complexes mixtes de chlorure d'aluminium et de titane ou d'aluminium et de silicium, ainsi que des sulfates ou lactates mixtes d'aluminium et de métal alcalin.

La concentration totale en les sels métalliques présents est de préférence comprise entre 0,05 et 0,5 atome de métal par litre de lotion, ou mieux entre 0,1 et 0.3. Le pH de cette solution ou suspension doit être de 2,5 à 9 et de préférence entre 3 et 8.

Cependant, le pH final, au moment de l'application de la composition A, ne doit pas dépasser 7 et il est de préférence compris entre 3 et 6. Pour cela, la solution ou suspension aqueuse, contenant les constituants $T_1$, $T_2$ et M, est additionnée d'une quantité suffisante d'un acide organique R, de façon à ajuster le pH à une telle valeur. L'acide organique R, soluble dans l'eau, peut être un acide linéaire ou ramifié, mono- ou polyacide, portant éventuellement une ou plusieurs fonctions alcool ou/et amine. On peut, bien entendu, employer conjointement plusieurs de tels acides. A titre d'exemple non limitatif, on peut citer les acides formique, acétique, propionique, citrique, lactique, tartrique, malique, amino-acétique, aspartique et glutamique.

De ce qui précède, on voit que la composition A, suivant l'invention, pour le traitement préalable des phanères, avant teinture, diffère beaucoup des solutions habituellement utilisées, notamment dans le cas de fourrures. En effet, au cours des travaux qui ont conduit à la présente invention, on a trouvé que, pour pouvoir fixer solidement et uniformément un colorant naturel sur une matière kératinique, il faut libérer la

surface de celle-ci non seulement de matières grasses, mais également de différentes autres substances, en particulier protéiniques, qui gênent la fixation du colorant. Ce but est atteint de façon inattendue, lorsqu'un tensio-actif dégraissant, notamment $T_1$ défini plus haut, est accompagné d'un second tensio-actif, également non ionique, $T_2$ possédant des groupements chimiques différents de ceux de $T_1$. En outre, contrairement à la plupart des solutions dégraissantes, connues, de caractère franchement basique, comme c'est le cas des savons classiques, la composition A, suivant l'invention, présente un pH ne dépassant pas 7, et elle est de préférence légèrement acide, ce qui est réalisé par l'adjonction d'un acide organique R hydrosoluble. Une autre différence réside dans la présence d'un sel métallique M qui rend la surface de la matière kératinique réceptive vis-à-vis des colorants naturels.

En définitive, la composition spéciale A, suivant l'invention, comprenant les substances décrites plus haut, $T_1+T_2+M+R$, constitue une combinaison nouvelle, qui améliore considérablement la teinture des phanères.

Constituants de la composition B

Le premier tensio-actif $T_3$ est du même type que la tensio-actif $T_1$ décrit plus haut: cela peut être le même composé ou un autre, appartenant à la même classe des éthers alkylaryliques polyalkoxylés. Sa concentration dans le liquide de teinture est généralement de 15 à 60 g/l ou mieux de 30 à 50 g/l.

$T_4$ peut être un tensioactif non ionique ou anionique; sa présence, sans être obligatoire, peut avoir une grande utilité pour exercer sur la matière kératinique traitée une ou plusieurs des actions, tels que nettoyage, surgraissage, facilité de peignage, gonflement, adoucissant, etc. Lorsque $T_4$ est non ionique, il peut être choisi parmi les différents composés tensio-actifs, décrits plus haut, à propos de $T_2$. Mais comme il peut également être anionique, il est possible de se servir d'un ou de plusieurs des agents suivants: alkyl sulfates, alkyl sulfonates, alkylaryl sulfonates, sulfates et/ou sulfonates d'amines grasses, acides amino acylés, par exemple sarcosine, huiles animales ou végétales sulfatées ou sulfonées, produits d'estérification d'alcool gras, alcool gras éthoxylés ou alkyl aryl éthoxylé, sulfoné. Cette énumération n'est donnée qu'à titre non limitatif; d'ailleurs, plusieurs agents tensio-actifs peuvent être utilisés conjointement. Leur concentration est en général de l'ordre de 5 à 30 g/l.

P, qui désigne un alcool, est un des constituants de la composition pour teinture suivant l'invention. Lorsque cet alcool ne porte qu'une fonction —OH, sa masse moléculaire doit être faible, autrement dit, il doit être en $C_1$ à $C_5$ et plus particulièrement éthanol, propanol-1, propanol-2, ou un des butanols-1, -2 ou 3. Cependant, conviennent également des polyalcools, en particulier les polyéthylène glycol, le polypropylène glycol, le glycérol, etc. Plusieurs alcools peuvent être utilisés conjointement, mais la concentration totale en ces constituants P doit être d'environ 10 à 50 g/l, avec préférence pour 15 à 35 g/l.

On a désigné, plus haut, par G, un autre constituant de la composition tinctoriale notamment un ou plusieurs acides gras ou savons alcalins de tels acides. Une des particularités de l'invention réside en effet en ce que la fixation de la teinture est améliorée par la présence des chaînes d'acides gras, c'est-à-dire d'acides carboxyliques en $C_8$ à $C_{22}$ et surtout en $C_{12}$ à $C_{18}$. Conviennent bien les différents acides gras courants, notamment laurique, myristique, palmitique, stéarique, arachidique, oléique, linoléique, linolénique, ricinoléique, etc. Ils peuvent donc être utilisés sous une ou plusieurs des formes courantes, c'est-à-dire à l'état de glycérides naturels, tels que graisses ou huiles, comme coprah, palmiste, palme, beurre de cacao, huile d'amande, d'olive, d'arachide, colza, maïs, sésame, tournesol, soja, pépins de raisin noix ou autres. Des savons correspondants, de sodium, potassium, ammonium, amines ou alcanolamines, peuvent également être employés, mais à des proportions telles que le pH de la composition soit inférieur à 8,5.

D'une façon générale, la concentration totale en les corps gras G, présents dans la teinture, est d'environ 5 à 40 g/l, les limites préférées étant de 15 à 30 g/l.

Le pH préféré de la solution ou suspension B pour teinture est de 6 à 8.

E désigne les différents adjuvants que l'on peut utilement incorporer à la solution de teinture, afin de lui communiquer la consistance et la viscosité appropriées. De tels adjuvants E sont généralement appelés épaississants. Ils sont connus dans l'art, on encite donc quelques-uns ici à titre d'exemple non limitatif. Polymères carboxyvinyliques, argiles, par exemple bentonites, montmorillonites. Dérivés de la cellulose, notamment carboxy-méthyl celluloses, méthyl celluloses, hydroxy éthyl ou hydroxy propyl celluloses. Polysaccharides, par exemple agar-agar, carraghénanes, alignates, dextranes, xanthanes, gommes de caroube ou guar, arabique ou adragante. Amidon et ses dérivés.

Un ou plusieurs de ces épaississants E peuvent être employés de façon à conférer à la solution ou suspension de teinture une viscosité appropriée. Lorsqu'il s'agit de traiter une chevelure sur tête, les viscosités les plus favorables sont comprises entre 0,500 et 2,500 Pas (500 et 2500 cp) et mieux entre 0,800 et 1,500 Pas (800 et 1500 cp) (viscosimètre Brookfield, mobile 3, à 60 tous/minute). Il est à noter qu'un avantage particulier résulte de l'emploi de ceux des épaississants qui confèrent à la solution un comportement pseudo plastique. La thixotropie du liquide permet, lors de la manipulation de la teinture, pour l'application, d'en faire abaisser fortement la viscosité par simple agitation; le liquide retrouve ensuite sa viscosité élevée dès que la manipulation cesse et la composition tient alors bien, d'elle-même, sur la chevelure. De telles compositions pseudo plastiques présentent des viscosités de 4,000 à 8,000 Pas (4000 et 8000 cp) pour 6 t/min et 0,500 à 2,500 Pas (500 à 2500 cp) pour 60 t/min.

(Viscosimètre Brookfield, Mobile n° 3).

C- Le constituant essentiel C, c'est-à-dire le ou les colorants voulus, est dissous ou dispersé dans la composition B décrite, de façon à obtenir l'intensité de coloration désirée. L'invention s'applique pratiquement à tous les colorants naturels qui peuvent être pris sous la forme d'un corps pur, isolé à partir d'un végétal, sous celle d'un extrait brut obtenu à partir du végétal ou bien sous la forme d'une poudre ou pâte formée par la division des parties de la plante, riches en le colorant donné; ainsi peut-on disperser dans la composition B une poudre de racines, de feuilles ou de bois, par exemple garence, henné ou santal rouge. La concentration en colorant C est naturellement très variable selon la nature du colorant, l'intensité de teinte voulue, et la capacité tinctoriale des phanères traités; cette grandeur est donc très variable. Cependant, dans la pratique courante, les concentrations vont le plus souvent de 0,2 à 200 g/l et, le plus souvant de 1 à 50 g/l.

Le tableau qui suit indique les noms des principaux colorants naturels convenant à la réalisation de l'invention. Ce tableau donne la désignation officielle selon le Color Index, les noms courants des plantes correspondantes, ainsi que ceux des colorants eux-mêmes fournis par ces plantes. Voir le Tableau à la page suivante.

Tableau de colorants naturels

| Color index | Noms courants principaux | Principes colorants principaux |
|---|---|---|
| JAUNE: | | |
| naturel 1 | Camomille des teinturiers, camomille allemande, camomille romaine (capitules floraux) | Apigénine |
| naturel 2 | Gaude—Réseda (toute la plante) | Lutéoline |
| naturel 6/19 | Safran (stigmates des fleurs) | Crocin, Crocétine |
| naturel 8 | Bois d'Arc (bois) | Morine |
| naturel 9 | Quercitron (écorce et bois) | Quercitrine |
| naturel 10 | Sophora Japonica (boutons floraux) | Rutine, Quercétine |
| naturel 11 | Mûrier des teinturiers (bois) | Maclurine |
| naturel 13 | Nerprun des teinturiers (baies) | Kampférol |
| naturel 14 | Bourdaine (écorce) | Emodine |
| naturel 23 | Rhubarbe de Chine (rhizome) | Chrysophanol |
| naturel 27 | Calendula, officinalis (souci) (fleurs) | Rubixanthine |
| naturel 28 | Kesu (fleurs séchées) | Butéine |
| naturel 3 | Curcuma (rhizome) | Curcumine |
| BRUN: | | |
| naturel 1 | Fustet, arbres à perruques, fustel jaune (écorce et bois) | Fisétine |
| naturel 3 | Acacia catechu, cachou, gambir noix d'Arec | Catéchine et tannins catéchiques |
| naturel 7 | Noyer (partie externe du fruit) | Juglone |
| ORANGE: | | |
| naturel 4 | Rocou (pulpe) | Bixine |
| naturel 6 | Henné (feuilles) | Lawsone |

Tableau de colorants naturels

| Color index | Noms courants principaux | Principes colorants principaux |
|---|---|---|
| BLEU: | | |
| naturel 1 | Indigo | Indigo |
| naturel 3 | Commeline | Awobanine |
| ROUGE: | | |
| naturel 8 | Garance (racine) | Alizarine, purpurine purpuroxanthine |
| naturel 9 | | |
| naturel 10 | | |
| naturel 11 et naturel 12 | | |
| naturel 18 | Morinda (racine) | Morindadiol |
| naturel 19 | | Morindone |
| naturel 20 | Orcanette (racine) | Alkanine |
| naturel 22 | Santal rouge (bois) | Santalines, santarubines |
| naturel 24 | Pernambouc, Sappan, Brésil, Bois rouge (bois) | Braziline, Brazileine |
| naturel 28 | Orseille, tournesol, litmus (traitement ammoniacal de lichen) | Dérivés de l'acide lecanorique |
| naturel 31 | Sang—Dragon (sécrétion des fruits) | Dracorhodine, Dracorubine |
| NOIR: | | |
| naturel 1 | Campêche, Bois bleu | Hematoxyline, Hematéïne |

Selon une forme d'exécution particulière de l'invention, dans des cas difficiles, notamment lorsqu'il s'agit de teindre une chevelure foncée, présentant des cheveux blancs, disséminés, de très bons résultats sont tout de même obtenus par l'adjonction d'un ou de plusieurs composés oxydants à la solution de traitement préalable A.

Cette forme d'exécution consiste à ajouter à la solution A des composés ayant la propriété d'être oxydants en milieu acide, plus particulièrement dans la zone de pH de 3 à 7; d'autre part, la solution ou suspension colorante doit contenir un composé porteur d'une ou de plusieurs fonctions éther et au moins une fonction alcool.

Les composés oxydants, additionnés à la solution de traitement préalable, peuvent être de diverses natures, minéraux ou organiques. Ils peuvent être des sels ou persels de cations alcalins comme perchlorates, perborates, persulfates, permanganates, chlorate, bromates, ou des peroxydes tels que peroxyde d'hydrogène ou de sodium, ou bien des peroxydes organiques comme, par exemple, le peroxyde d'urée, de benzoyle ou de succinyle.

La concentration en agent oxydant est comprise entre 2 et 30 g/l et de préférence entre 5 et 15 g/l.

Les composés présentant une ou plusieurs fonctions éther et au moins une fonction alcool libre répondent à la forme générale:

$$Z—(CH_2)_n—[OR—]_mOH \qquad I$$

où Z est un atome d'hydrogène, un alkyle en $C_1$ à $C_4$, un OH ou un R'—OOC— dont le R' est un alkyle en $C_1$ à $C_{18}$, hydroxy-alkyle ou glycéryle, n étant 1 à 18 et m 1 à 80, tandis que R désigne un polyalkylène pouvant être ramifié, en particulier

$$-CH_2CH_2- \quad ou \quad -CH(CH_3)-CH_2-.$$

Une forme particulière d'un tel alcool-éther peut être représentée par la formule:

$$HO-R^1-O-R^2-OH \qquad II$$

dans laquelle $R^1$ est un reste de mono- ou polyalcool ou bien un ester d'acide gras et de polyol. Ainsi $R^1$ peut-il être un reste de mono- ou polyalcool en $C_3$ à $C_{18}$, par exemple d'alcool butylique, pentylique, hexylique, caprique, laurique, décylique, myristique, palmitique, stéarique, cétylique, isostéarique, oléique, butanediol, propanediol, hexanediol, glycérol, etc.; il peut également être constitué par un ester de glycéryle, tel que, par exemple, caprate, caprylate, laurate, myristate, oléate, cocoate, stéarate et isostéarate.

$R^2$ est un reste de polyalcool ou de polyalkylène glycol, comme par exemple polyéthylène glycol ou/et polypropylène glycol, dans lesquels le nombre de motifs alkylène glycol est généralement compris entre 3 et 80 et plus particulièrement entre 5 et 50.

La concentration en composés de formule I ou II est généralement comprise entre 5 et 200 g/l et de préférence entre 10 et 100 g/l.

Voici quelques exemples non limitatifs pour illustrer l'invention.

Exemples 1 à 5

Compositions A pour le traitement préalable des cheveux ou poils

Exemple 1

$T_1$—4,5 parties en poids de nonyl phénol condensé avec 6 moles d'oxyde d'éthylène;

$T_2$—0,5 partie de stéarate polyoxyéthyléné à 40 moles d'oxyde d'éthylène +1,2 partie de diéthanolamide de l'huile de coprah;

M—3,53 parties de lactate d'aluminium;

R—quantité suffisante d'acide acétique pour porter la solution au pH de 3,5;
90,27 parties d'eau désionisée.

Exemple 2

$T_1$—3 parties d'octyl phénol condensé avec 6 moles de $(CH_2)_2O$+2 parties de nonyl phénol condensé avec 12 moles de $(CH_2)_2O$;

$T_2$—1 partie de stéarate de polyoxyéthylène glycol à 40 moles d'oxyde d'éthylène;

M—5,16 parties de sulfate double d'aluminium et de potassium;

R—acide citrique dans une quantité d'eau désionisée pour un total de 100 parties: pH 3.

Exemple 3

$T_1$—5,2 parties de nonyl phénol condensé avec 10 moles d'oxyde d'éthylène;

$T_2$—0,8 partie de stéarate de polyoxyéthylène à 20 moles d'oxyde d'éthylène;

M—2,28 parties d'hydroxychlorure d'aluminium en solution aqueuse à 50%, le rapport Al/Cl étant d'environ 2;

R—acide tartrique pour avoir pH 4 dans 100 parties de solution faite avec de l'eau désionisée.

Exemple 4

$T_1$—5 parties d'octyl phénol à 11 moles d'oxyde d'éthylène;

$T_2$—0,4 partie stéarate de polyoxyéthylène à 30 $(CH_2)_2O$+0,4 partie de mono éthanolamide de coprah condensé avec 10 moles de $(CH_2)_2O$;

M—1,2 partie d'hydroxychlorure d'aluminium cristallisé+0,57 partie de $MgCl_2 \cdot 6H_2O$;

R—acide citrique pour pH 4 eau désionisée, quantité suffisante pour 100 parties de solution.

Exemple 5

$T_1$—3 parties de nonyl phénol à 10 moles de $(CH_2)_2O$+2,5 parties de nonyl phénol avec 12 moles de $(CH_2)_2O$;

$T_2$ 0,8 partie de di-éthanolamide de l'huile de coprah;

M—3,3 parties d'acétate de zinc;

R—acide lactique pour pH 3,5 eau désionisée pour 100 Parties de mélange.

Exemples 6 à 10

Compositions B de base support de la teinture

Dans ces exemples, les quantités des composants sont indiqués en % en poids de la solution formée avec de l'eau désionisée.

Exemple 6

$T_3$—3,2 de nonyl-phénol à 6 moles d'oxyde d'éthylène et 0,8 de mono oléate de sorbitan;

$T_4$—0,6 de sulfonate de dodécyl benzène;

P—1,7 isopropanol;

G—2,0 acide stéarique;

E—1,0 bentonite

0,26 de NaOH.

La bentonite est d'abord mouillée à l'isopropanol, puis incorporée sous agitation dans une fraction de l'eau à laquelle on ajoute ensuite l'hydroxyde de sodium. D'autre part, on mélange les divers tensio-actifs avec le reste d'eau et l'acide stéarique, puis on incorpore le tout dans la fraction contenant la bentonite.

Exemple 7

$T_3$—3,5 nonyl phénol à 10 moles d'oxyde d'éthylène;

$T_4$—0,9 alcool laurique condensé avec 23 moles $(CH_2)_2O$+0,7 stéarate de polyoxyéthylène à 40 moles $(CH_2)_2O$;

P—1,5 isopropanol;

G—1,7 acide linoléique;

E—1,5 alginate de sodium de haute viscosité.

Dans cette préparation l'alginate est d'abord mélangé avec l'isopropanol, puis ajouté à une fraction de l'eau, sous agitation. D'autre part, on mélange les tensio actifs et les colorants avec de l'eau et avec l'acide linoléique: le mélange obtenu est incorporé dans la solution d'alginate. On trouve alors au Viscosimètre Brookfield, avec le Mobile 3, 2,500 Pas (2500 cp) à 6 t/min et 0,850 Pas (850 cp) à 60 t/min.

Exemple 8

$T_3$—0,8 octyl phénol à 6 $(CH_2)_2O$+3,0 nonyl phénol à 10 $(CH_2)_2O$;

$Y_4$—0,7 diéthanolamide de coprah à 10 moles d'oxyde d'éthylène+1,0 lauryl sulfate de triéthanolamine;

P—2,5 isopropanol;

G—0,5 d'acide oléique+2,5 d'huile d'olive;

E—1,0 carboxyméthyl cellulose, haute viscosité 0,75 triéthanolamine.

L'effet thixotropique de la carboxyméthyl cellulose est obtenu par un mode opératoire similaire à celui qui a été appliqué à l'alginate de sodium dans l'exemple précédent. La solution de teinture obtenue présente une viscosité de 4,000 Pas (4000 cp) à 6 t/min et 0,840 Pas (840 cp) à 60 t/mn.

Exemple 9

$T_3$—3,0 nonyl phénol à 12$(CH_2)_2O$;

$T_4$—0,8 alcool cétylique à 20$(CH_2)_2O$;

P—2,0 isopropanol;

G—2,2 oléate de sodium;

E—1,0 gomme xanthane (Keltrol R de la Société Kelco).

Pendant la préparation de cette teinture, la gomme xanthane est d'abord mouillée avec l'isopropanol, et le mélange obtenu est incorporé dans l'eau contenant tous les autres ingrédients. La viscosité est de 5,600 Pas (5600 cp) à 6 t/min et de 0,550 Pas (550 cp) à 60 t/min.

Exemples 10 à 17

Réalisation de teintures.

Exemple 10

Sur des cheveux châtain clair on applique la composition A n° 1 en massant légèrement pour bien répartir le produit.

Après 5 minutes, les cheveux sont rincés à l'eau tiède. On applique ensuite la composition B n° 6 à laquelle sont ajoutés 8 ml d'une dispersion d'un extrait de garance dans le propylène glycol.

Après 30 minutes, les cheveux sont rincés à l'eau tiède puis séchés. Ils sont alors d'une belle couleur acajou cuivrée brillante.

Ce temps de pause de la teinture peut se dérouler soit à l'air libre soit sous une coiffe destinée à empêcher la sensation de froid due à l'évaporation de l'eau.

Exemple 11

Selon le mode opératoire de l'exemple 10, on utilise successivement la composition A no° 2 et la composition B n° 7.

Dans cette dernière on incorpore 5 ml d'une solution hydroalcoolique d'un extrait de curcuma.

Les cheveux châtain clair sont devenus d'un blond doré lumineux.

Exemple 12

Sur des cheveux châtains préalablement traités avec la composition A n° 4 on dépose la composition B n° 8 dans laquelle on dissout 0,2 g de lawsone. Après séchage, les cheveux sont d'un bel acajou.

Exemples 13 à 17

Dans ces exemples, on utilise les présentations particulières, suivant l'invention, d'extraits colorants décrits dans les pages 5 et 6 plus haut.

Ces extraits sont incorporés directement dans la composition B correspondante, comme le montre le Tableau à la page 10.

Exemple 18

Cas d'une chevelure brune avec des cheveux blancs, disséminés.

Deux portions d'une solution de traitement préalable sont préparées suivant l'exemple 4. A l'une d'elles on ajoute 1,2 parties de persulfate de sodium.

D'autre part, on prépare deux portions de solution de teinture selon l'exemple 9. Une d'elles est additionnée de 5 parties d'un polyéther résultant de la réaction de 1 mole d'alcool myristique $CH_3(CH_2)_{13}OH$ avec 5 moles d'oxyde de propylène et 5 moles d'oxyde d'éthylène. Dans chacun de ces deux solutions de teinture on incorpore un mélange contenant 0,5 g d'extrait de Campèche et 1,5 g d'extrait de bois rouge.

Sur une moitié d'une chevelure brune, présentant des cheveux blancs disséminés, on applique, selon le procédé décrit à l'exemple 1, les préparations ci-dessus, ne contenant pas les additifs; sur l'autre moitié sont appliquées les préparations contenant ces additifs.

Dans le premier cas, on obtient une coloration brun-café intense. La différence entre cheveux bruns et blancs est nettement moins visible qu'avant teinture, mais elle n'est qu'incomplètement estompée.

Dans le second cas, on obtient également la coloration bruncafé intense; mais les cheveux blancs ne sont plus visibles.

TABLEAU

| | Exemple 13 | Exemple 14 | Exemple 15 | Exemple 16 | Exemple 17 |
|---|---|---|---|---|---|
| Composition A | n° 3 | n° 5 | n° 2 | 1 | 1 |
| Composition B | n° 9 | n° 8 | n° 6 | 7 | 7 |
| Extraits colorants | Campêche: 0,4 g | Campêche: 0,5 g | Santal rouge: 0,8 g Rhubarbe de Chine: 1 g | Bois d'arc: 1 g Noyer: 0,5 g | Campêche: 0,6 g |
| Cheveux d'origine | châtain foncé +cheveux blancs | blond | blond | châtain clair | blond clair |
| Teintes obtenues | noir brillant bleuté | brun-marron | châtain doré cuivré | châtain foncé doré | noir bleuté |

**Revendications**

1. Procédé de coloration de phanères avec des colorants naturels au moyen d'une solution ou dispersion aqueuse renfermant un agent tensioactif, caractérisé en ce qu'il est effectué en deux étapes successives, dont la première consiste en un traitement des phanères avec une solution renfermant au moins deux composés tensioactifs, non ioniques, de nature chimique différente, et un ou plusieurs sels de métaux des groupes II à V de la Classification Périodique des Eléments, sans colorant, tandis que la seconde étape comprend l'application aux phanères d'une solution ou dispersion qui contient le ou les colorants naturels, au moins un composé tensioactif non ionique, un ou plusieurs agents tensioactifs non ioniques et/ou anioniques, un alcool en $C_1$ à $C_5$, un glycol ou glycérol et un acide gras en $C_8$ à $C_{22}$, un glycéride ou savon d'un tel acide gras.

2. Procédé suivant la revendication 1, caractérisé en ce que le traitement de la première étape est effectué en milieu dont le pH ne dépasse pas 7 et est, de preférencé, de 3 à 6, tandis que le liquide, employé pour la seconde étape, présente un pH inférieur à 8,5, de préférence compris entre 6 et 8.

3. Procédé suivant la revendication 1 ou 2, caractérisé en ce que la solution ou dispersion, utilisée dans la deuxième étape contient un épaississant, de façon à ce que sa viscosité, mesurée au viscosimètre Brookfield (avec le mobile n° 3) soit d'environ 0,500 à 2,500 Pas (500 à 2500 cp) pour 60 tours/min, et de préférence 0,800 à 1,500 Pas (800 à 1500 cp).

4. Procédé suivant la revendication 3, caractérisé en ce qu'un ou plusieurs des épaississants employés rendent la solution ou dispersion tixotropique, lui conférant une viscosité préférée de 4,000 à 8,000 Pas (4000 à 8000 cp) à 6 tours/min au viscosimètre Brookfield (mobile n° 3).

5. Procédé suivant une des revendications précédentes, caractérisé en ce que le traitement de la première étape est effectué en environ 3 à 30 minutes et celui de la seconde étape en 10 à 60 minutes.

6. Procédé suivant une des revendications précédentes, caractérisé en ce que la première étape est effectuée avec la solution sans colorant, à laquelle on ajoute préalablement un persel ou peroxyde inorganique ou un peroxyde organique, tandis que la solution ou dispersion colorante servant à la deuxième étape, renferme un composé

$$Z—(CH_2)_n—(OR)_mOH$$

où Z est un atome de H, un alkyle en $C_1$ à $C_4$, un OH ou un R'OOC dont le R' est un alkyle en $C_1$ à $C_{18}$, hydroxyalkyle ou glycéryle, n étant 1 à 18 et m 1 à 80, alors que R désigne un polyalkylène pouvant être ramifié.

7. Composition pour la réalisation de la première étape du procédé suivant une des revendications 1 à 6, comprenant une solution aqueuse d'un agent mouillant ou détergent, caractérisée en ce qu'elle renferme à la fois: un ou plusieurs composés tensioactifs ($T_1$) alkyl aryl polyalcoxylés, au moins un autre composé tensioactif ($T_2$) non ionique, sans groupes aryliques, un sel non toxique (M) de métal des groupes II à V de la Classification Périodique des Eléments ainsi qu'une quantité suffisante d'un acide organique (R) soluble dans l'eau, pour que le pH de la composition ne dépasse pas 7 et soit, de préférence, compris entre 3 et 6.

8. Composition suivant la revendication 7, caractérisée en ce qu'elle renferme, par litre: 20 à 80 g d'un ($T_1$) alkyl phénol polyéthoxylé

$$R'—C_6H_4—O(CH_2CH_2O)_nH$$

où R' est un alkyle en $C_1$ à $C_{20}$ et n=5 à 15; 5 à 30 g d'un tensioactif non ionique sans groupes aryliques ($T_2$); 0,05 à 0,5 atome d'Al, pris sous la forme d'un sel (M) non toxique, et une quantité suffisante d'un acide organique (R), aliphatique en $C_2$ à $C_6$, pour ajuster le pH de la composition à une valeur de 3 à 6.

9. Composition pour la réalisation de la seconde étape du procédé suivant une des revendications 1 à 6, comprenant une solution ou dispersion aqueuse d'un colorant naturel et au moins d'un agent tensioactif, caractérisée en ce qu'elle contient à la fois: un ou plusieurs composés tensioactifs ($T_3$) alkyl aryl polyalcoxylés; au moins un autre composé tensioactif ($T_4$) non ionique, sans groupes aryliques, ou bien anionique; un alcool (P) en $C_1$ à $C_5$ ou un polyalcool glycolique ou glycérique; un acide gras en $C_8$ à $C_{22}$, ester ou savon d'un tel acide (G), et de préference également un épaississant (E) minéral ou organique, notamment argile, dérivé cellulosique, polysaccharide, gomme ou amidon, le pH de la composition étant de 6 à 8.

10. Composition suivant la revendication 9, renfermant par litre 1 à 200 g de colorant, caractérisée en ce qu'elle contient en même temps, par litre: 15 à 60 g d'un ($T_3$) alkyl phénol polyéthoxylé

$$R'—C_6H_4—O(CH_2CH_2O)_nH$$

où R' est un alkyle en $C_1$ à $C_{20}$ et n=5 à 15; 0 à 30 g d'un composé ($T_4$) tensioactif non ionique, non arylique, ou bien anionique, notamment sulfate ou sulfonate; 10 à 50 g d'un alcool (P) en $C_1$ à $C_5$ ou d'un polyol dérivé d'un alcool en $C_2$ ou $C_3$; 5 à 40 g d'un acide gras en $C_8$ à $C_{22}$, d'un ester ou d'un savon d'un tel acide (G); ainsi qu'une quantité d'épaississant telle que la composition présente une viscosité de 0,500 à 2,500 Pas (500 à 2500 cp) au viscosimètre Brookfield pour 60 tours/min (mobile n° 3).

11. Composition suivant la revendication 10, caractérisée en ce que l'épaississant est une substance lui conférant la thixotropie, en particulier bentonite, alginate de Na, carboxyméthyl-cellulose, ou gomme xanthane, au point d'élever la viscosité de la composition à une valeur de 4,000 à 8,000 Pas (4000 à 8000 cp) pour 6 tours/min (viscosimètre Brookfield avec mobile n° 3).

12. Composition suivant la revendication 7 ou 8, caractérisée en ce qu'elle contient un persel ou peroxyde inorganique ou un peroxyde organique.

13. Composition suivant une des revendications 9 à 11, caractérisée en ce qu'elle contient un composé

$$Z—(CH_2)_n—(OR)_mOH$$

où Z est un atome de H, un alkyle en $C_1$ à $C_4$, un ou un R'OOC dont le R' est un alkyle en $C_1$ à $C_{18}$, hydroxyalkyle ou glycéryle, n étant 1 à 18 et m 1 à 80, alors que R désigne un polyalkylène pouvant être ramifié, à raison de 5 à 200 g par litre.

**Patentansprüche**

1. Verfahren zur Färbung von Oberhautanhangsgebilden mit natürlichen Farbstoffen, mittels einer wässerigen Lösung oder Dispersion, die ein oberflächenaktives Mittel enthält, dadurch gekennzeichnet, dass es in zwei aufeinanderfolgenden Stufen durchgeführt wird, von denen die erste in einer Behandlung der Oberhautanhangsgebilde mit einer Lösung besteht, die mindestens zwei oberflächenaktive, nichtionische, in ihrer chemischen Natur unterschiedliche oberflächenaktive Mittel, und ein oder mehrere Salze von Metallen der Gruppen II bis V des Periodensystems der elemente, ohne Farbstoffe enthält, während die zweite Stufe den Auftrag einer Lösung oder einer Dispersion auf die Oberhautanhangsgebilde umfasst, die den oder die natürliche(n) Farbstoff(e), mindestens eine nicht-ionische oberflächenaktive Verbindung und ein oder mehrere nicht-ionische und/oder anionische oberflächenaktive Mittel, einem $C_1—C_5$ Alkohol, ein Glykol oder Glycerin und eine $C_8—C_{22}$ Fettsäure, ein Glycerid oder eine Seife einer solchen Fettsäure enthält.

2. Verfahren nach Anspruch 1, dadurch gekennzeichnet, dass die Behandlung in der ersten Stufe in einem Medium durchgeführt wird, dessen pH-Wert 7 nicht überschreitet, und vorzugsweise 3 bis 6 ist, während die Flüssigkeit, die für die zweite Stufe verwendet wird, einen pH-Wert unter 8,5, vorzugsweise zwischen 6 und 8, aufweist.

3. Verfahren nach Anspruch 1 oder 2, dadurch gekennzeichnet, dass die in der zweiten Stufe verwendete Lösung oder Dispersion ein Verdickungsmittel enthält, derart, dass ihre Viskosität, gemessen mit dem Brookfield Viskosimeter (mit der Spindel Nr. 3), bei etwa 0,500 bis 2,500 Pa s (500 bsi 2500 cP) bei 60 Upm und vorzugsweise bei 0,800 bis 1,500 Pa s (800 bis 1500 cP) liegt.

4. Verfahren nach Anspruch 3, dadurch gekennzeichnet, dass ein oder mehrere der verwendeten Verdickungsmittel die Lösung oder Dispersion thixotrop machen und ihr eine Viskosität von vorzugsweise 4,000 bis 8,000 Pa s (4000 bis 8000 cP) bei 6 Upm im Brookfield Viskosimeter (Spindel Nr. 3) verleihen.

5. Verfahren nach einem der vorhergehenden Ansprüche, dadurch gekennzeichnet, dass die Behandlung der ersten Stufe etwa 3 bis 30 min und die der zweiten Stufe 10 bis 60 min durchgeführt wird.

6. Verfahren nach einem der vorhergehenden Ansprüche, dadurch gekennzeichnet, dass die erste Stufe mit der Lösung ohne Farbstoff, der man vorher ein Persalz oder anorganisches Peroxyd oder organisches Peroxyd zufügt, durchgeführt wird, während die färbende Lösung oder Dispersion, die der zweiten Stufe dient, eine Verbindung

$$Z—(CH_2)_n—(OR)_mOH$$

enthält, worin Z ein H-Atom, ein $C_1—C_4$ Alkyl, ein OH oder ein R'OOC ist, dessen R' ein $C_1—C_{18}$ Alkyl, Hydroxyalkyl oder Glyceryl ist, n 1 bis 18 und m 1 bis 80 ist, während R ein Polyalkylen bedeutet, das verzweigt sein kann.

7. Zusammensetzung zur Durchführung der ersten Stufe des Verfahrens nach einem der Ansprüche 1 bis 6, enthaltend eine wässerige Lösung eines Benetzungsmittels oder Detergens, dadurch gekennzeichnet, dass sie gleichzeitig enthält: eine oder mehrere oberflächenaktive polyalkoxylierte Alkylaryl-Verbindungen ($T_1$), mindestens eine andere nicht-ionische oberflächenaktive Verbindung ($T_2$) ohne Arylgruppen, ein nicht-toxisches Salz (M) von einem Metall der Gruppen II bis V des Periodensystems der Elemente, sowie eine ausreichende Menge einer wasserlöslichen organischen Säure (R), so dass der pH-Wert der Zusammensetzung 7 nicht überschreitet und vorzugsweise bei 3 bis 6 liegt.

8. Zusammensetzung nach Anspruch 7, dadurch gekennzeichnet, dass die pro Liter enthält: 20 bis 80 g eines polyethoxylierten Alkylphenols ($T_1$)

$$R'—C_6H_4—O(CH_2CH_2O)_nH,$$

worin R' ein Alkyl mit $C_1$ bis $C_{20}$ ist und n=5 bis 15; 5 bis 30 g eines nicht-ionischen, oberflächenaktiven Mittels ($T_2$) ohne Arylgruppen; 0,05 bis 0,5 Atome von Al, in der Form eines nicht-toxischen Salzes (M), eine

ausreichende Menge einer aliphatischen $C_2$—$C_6$ Säure (R), um den pH-Wert der Zusammensetzung auf einen Wert von 3 bis 6 einzustellen.

9. Zusammensetzung zur Durchführung der zweiten Stufe des Verfahrens gemäss einem der Ansprüche 1 bis 6, enthaltend eine wässerige Lösung oder Dispersion eines natürlichen Farbstoffs und mindestens ein oberflächenaktives Mittel, dadurch gekennzeichnet, dass sie gleichzeitig enthält: eine oder mehrere oberflächenaktive polyalkoxylierte Alkylaryl-Verbindungen ($T_3$); mindestens eine andere oberflächenaktive nicht-ionische, nicht-arylische oder aber anionische oberflächenaktive Verbindung ($T_4$); einen Alkohol (P) mit $C_1$ bis $C_5$ oder eine glykolischen oder glycerinischen Polyalkohol; eine Fettsäure mit $C_8$ bis $C_{22}$, einen Ester oder eine Seife einer derartigen Säure (G), und vorzugsweise auch ein Verdickungsmittel (E), das mineralisch oder organisch, insbesondere eine Tonerde, ein Cellulosederivat, Polysaccharid, Gummi oder Stärke ist, wobei der pH-Wert der Zusammensetzung 6 bis 8 ist.

10. Zusammensetzung nach Anspruch 9, enthaltend pro Liter 1 bis 200 g Farbstoff, dadurch gekennzeichnet, dass sie gleichzeitig pro Liter enthält: 15 bis 60 g eines polyethoxylierten Alkylphenols ($T_3$)

$$R'—C_6H_4—O(CH_2CH_2O)_nH,$$

worin R' ein Alkyl mit $C_1$ bis $C_{20}$ ist und n=5 bis 15; 0 bis 30 g einer nicht-ionischen, nicht-arylischen oder aber anionischen oberflächenaktiven Verbindung ($T_4$), insbesondere ein Sulfat oder Sulfonat; 10 bis 50 g eines $C_1$ bis $C_5$ Alkohols (P) oder eines von einem $C_2$ oder $C_3$ Alkohol abgeleiteten Polyol; 5 bis 40 g einer Fettsäure mit $C_8$ bis $C_{22}$, einem Ester oder einer Seife einer derartigen Säure (G); sowie eine derartige Menge an Verdickungsmittel, dass die Zusammensetzung eine Viskosität von 0,500 bis 2,500 Pa s (500 bis 2500 cP) mit dem Brookfield Viskosimeter bei 60 Upm (Spindel Nr. 3) aufweist.

11. Zusammensetzung nach Anspruch 10, dadurch gekennzeichnet, dass das Verdickungsmittel eine Substanz ist, die ihr Thixotropie verleiht, insbesondere Bentonit, Na-Alginat, Carboxymethyl-cellulose, oder Xanthangummi, um die Viskosität der Zusammensetzung auf einen Wert von 4,000 bis 8,000 Pa s (4000 bis 8000 cP) bei 6 Upm (Brookfield Viskosimeter mit Spindel Nr. 3) anzuheben.

12. Zusammensetzung nach Anspruch 7 oder 8, dadurch gekennzeichnet, dass sie ein Persalz oder ein anorganisches Peroxyd oder eine oxidierende Verbindung enthält.

13. Zusammensetzung nach einem der Ansprüche 9 bis 11, dadurch gekennzeichnet, dass sie eine Verbindung,

$$Z—(CH_2)_n—(OR)_mOH$$

enthält, worin Z ein H-Atom, ein $C_1$—$C_4$ Alkyl, ein OH oder ein R'OOC ist, dessen R' ein $C_1$—$C_{18}$ Alkyl, Hydroxyalkyl oder Glyceryl ist, n 1—18 und m 1 bis 80 ist, während R ein Polyalkylen bedeutet, das verzweigt sein kann, in einer Menge von 5 bis 200 g pro Liter enthält.

**Claims**

1. Process of coloration of phanera with natural colorants by means of an aqueous solution or dispersion containing a surfactant, characterised in that it is carried out in two successive stages, of which the first comprises treatment of the phanera with a solution containing at least two non-ionic surfactants of different chemical natures and one or more salts of metals of groups II to V of the periodic classification of the elements, without colorant, whilst the second stage comprises the application to the phanera of a solution or dispersion which contains the natural colorant(s), at least one non-ionic surfactant, one or more non-ionic and/or anionic surfactants, a $C_1$ to $C_5$ alcohol, a glycol or glycerol and a $C_8$ to $C_{22}$ fatty acid, or glyceride or soap of such a fatty acid.

2. Process according to claim 1, characterized in that the first stage of treatment is carried out in a medium of which the pH does not exceed 7 and which is, preferably, from 3 to 6, whilst the liquid used in the second stage has a pH less than 8.5, preferably between 6 and 8.

3. Process according to claim 1 or 2, characterised in that the solution or dispersion used in the second stage contains a thickener, so that its viscosity, measured by a Brookfield viscometer (with mobile n° 3) is in the region of 0.500 to 2.500 Pas (500 to 2500 cp) at 60 rev/min, and preferably 0.800 to 1.500 Pas (800 to 1500 cp).

4. Process according to claim 3, characterised in that one or more of the thickeners used renders the solution or dispersion thixotropic, conferring on it a viscosity preferably of 4.000 to 8.000 Pas (4000 to 8000 cp) at 6 rev/min in a Brookfield viscometer (mobile n° 3).

5. Process according to any of the preceding claims, characterised in that the first stage of treatment is carried out for about 3 to 30 minutes and the second stage for 10 to 60 minutes.

6. Process according to any of the preceding claims, characterised in that the first stage is carried out with the solution without colorant, to which a persalt or inorganic peroxide or an organic peroxide has previously been added, whilst the coloured solution or dispersion used in the second step contains a compound

$$Z—(CH_2)_n—(OR)_mOH$$

where Z is an hydrogen atom, a $C_1$ to $C_4$ alkyl group, an OH group or R'OOC of which R' is a $C_1$ to $C_{18}$ alkyl group, an hydroxyalkyl group or a glyceryl group, n being 1 to 18 and m 1 to 80, whilst R designates a polyalkylene which may be branched.

7. Composition for carrying out the first stage of the process according to any of claims 1 to 6, comprising an aqueous solution of a wetting agent or detergent, characterised in that it also contains: one or more polyalkoxylated alkyl aryl surfactants ($T_1$), at least one other non-ionic surfactant ($T_2$) without aryl groups, a non-toxic salt (M) of a metal of groups II to V of the periodic classification of the elements, as well as a sufficient quantity of a water-soluble organic acid (R) for the pH of the composition not to exceed 7 and preferably to be between 3 and 6.

8. Composition according to claim 7, characterised in that it contains, per litre: 20 to 80 g of a polyethoxylated alkyl phenol ($T_1$)

$$R'-C_6H_4-O(CH_2CH_2O)_nH$$

where R' is a $C_1$ to $C_{20}$ alkyl group and n=5 to 15; 5 to 30 g of a non-ionic surfactant without aryl groups ($T_2$); 0.05 to 0.5 atom of Al, in the form of a non-toxic salt (M), and a sufficient quantity of a $C_2$ to $C_6$ aliphatic organic acid (R) to adjust the pH of the composition to a value of 3 to 6.

9. Composition for carrying out the second stage of the process according to any of claims 1 to 6, comprising an aqueous solution or dispersion of a natural colorant and at least one surfactant, characterised in that it also contains: one or more polyalkoxylated alkyl aryl surfactants ($T_3$); at least one other non-ionic, without aryl groups, or anionic surfactant ($T_4$), a $C_1$ to $C_5$ alcohol (P) or a glycolic or glyceryl polyalcohol; a $C_8$ to $C_{22}$ fatty acid or an ester or soap of such an acid (G), and preferably also a mineral or organic thickener (E), particularly a clay, a cellulosic derivative, polysaccharide, gum or starch, the pH of the composition being from 6 to 8.

10. Composition according to claim 9, containing 1 to 200 g of colorant per litre, characterised in that it also contains, per litre: 15 to 60 g of a polyethoxylated alkyl phenol ($T_3$)

$$R'-C_6H_4-O(CH_2CH_2O)_nH$$

where R' is a $C_1$ to $C_{20}$ alkyl group and n=5 to 15; 0 to 30 g of a non-ionic non-aryl, or anionic, surfactant ($T_4$), particularly a sulphate or sulphonate; 10 to 50 g of a $C_1$ to $C_5$ alcohol (P) or a polyol derivative of a $C_2$ or $C_3$ alcohol; 5 to 40 g of a $C_8$ to $C_{22}$ fatty acid, or an ester or a soap of such an acid (G); as well as a quantity of thickener such that the composition has a viscosity of 0.500 to 2.500 Pas (500 to 2500 cp) on a Brookfield viscometer for 60 rev/min (mobile n° 3).

11. Composition according to claim 10, characterised in that the thickener is a substance providing thixotropy, in particular bentonite, sodium alginate, carboxymethylcellulose, or xanthane gum, in such an amount as to raise the viscosity of the composition to a value of 4.000 to 8.000 Pas (4000 to 8000 cp) for 6 rev/min (Brookfield viscometer with mobile n° 3).

12. Compound according to claim 7 or 8, characterised in that it contains a persalt, an inorganic peroxide or an organic peroxide.

13. Composition according to any of claims 9 to 11, characterised in that it contains a compound

$$Z-(CH_2)_n-(OR)_mOH$$

where Z is a hydrogen atom, a $C_1$ to $C_4$ alkyl group, an OH group or an R'OOC group of which R' is a $C_1$ to $C_{18}$ alkyl group, an hydroxyalkyl group or a glyceryl group, n being 1 to 18 and m 1 to 80, whilst R represents a polyalkylene, which may be branched, in an amount of 5 to 200 g per litre.